# EUROPEAN PATENT APPLICATION

(11) **EP 2 381 376 A1**
(43) Date of publication of application: **26.10.2011**
(21) Application number: 10160726.5
(22) Date of filing: 22.04.2010
(51) Int. Cl.: G06F 19/00

(54) **Peritoneal dialysis prescription system and method**

(71) Applicant: DEBIOTECH S.A., 1004 Lausanne (CH)
(72) Inventor: Neftel, Frédéric, 1005, Lausanne (CH)
(74) Representative: Grosfillier, Philippe

(57) **Abstract**

Method for prescribing a peritoneal dialysis treatment comprising the following steps :
- collecting patient specific data,
- determining at least one target,
- defining a series of values of the type [V;t] which allow to achieve said target wherein V represents the injected volume of dialysate and t the duration of treatment, based on said patient specific data,
- displaying said series of values on a map.

The invention also relates to a system using this method.

## Description

### Field of invention

The invention relates to dialysis, such as peritoneal dialysis. It more precisely concerns the prescription of such a treatment.

### State of the art

Prescription of a therapy for a patient is currently done, e.g. for peritoneal dialysis, by use of simulation tools giving a treatment result based on a certain prescription model. Such tools are using patient membrane characteristics (resulting from a PET test or, preferably, a PDC test) as basis for their calculation and require the prescription giver (doctor or nurse) to input a given therapy (such as a night APD exchange, fixing the number of liters and duration as well as the glucose concentration) in order to calculate the result (e.g. in terms of UF, urea clearance and/or sodium removal). The prescription giver (doctor or nurse) will need to compare several possible treatments (e.g. different glucose concentrations, durations, volumes), usually one by one, in order to find the most appropriate treatment for the patient. Examples of such simulations tools are PD Adequest, from Baxter or Synergy, from Gambro. As such, those system are not realy simple to use nor very precise in the indication given to the prescription giver toward the best treatment for the patient considered, despite the amount of information available from such patient (such as PDC test results).

### General description of the invention

The present invention offers an efficient, precise, fast and user-friendly prescription for a dialysis treatment, such as for peritoneal dialysis.

It concerns a method and a system as defined in the claims.

The target prescription allows the user of the dialysis prescription system to simultaneously visualize and evaluate different criteria to quantify the specific outcome for a patient of a peritoneal dialysis session by means of a single interactive chart and determine the best parameters to be selected for such therapy to obtain a given result.

As claimed, the invention consists of a computing device that is programmed to compute the specific outcome of peritoneal dialysis therapy for a given patient, based on its own membrane or filtration characteristics, and to display simultaneously on a single chart one or several outcomes for multiple therapies differing by total duration and total volume of dialysate injected and/or dialysate fluid (such as glucose concentration). This amount of data is made directly interpretable by the user who can see the isolines (the set of points on the map sharing equal outcome values) of different possible targets to evaluate the performance of a therapy, such as ultrafiltration, clearances of molecules, sodium removal, glucose absorbtion etc. Such maps are preferably made interactive with the user by highlighting the isoline corresponding to the specific target value selected by him. This allows the user to select the most convenient combination of time and fluid volume, or glucose concentration, to achieve a desired clearance target or ultrafiltration target for a specific patient. For a more advanced use of this functionality, the maps of two specified targets can be superposed (by superposing the isolines of one and the surface plots of the other). These maps are advantageously accompanied by a side cursor to select the glucose concentration associated with the dialysate bags or another parameter of interest (such as volume of fluid, time of treatment or additional day exchange effect).

An additional information can be displayed in the background, choosing different colors or intensities corresponding to different region of values (e.g. for sodium absorbtion, ultrafiltration, glucose absorbtion, clearance of creatinine or urea, etc..) so that the user can visualize the consequence of its target choice on the additional information of interest or restrict his choice to the region of the additional information corresponding to the best one for the patient in order to select the appropriate target value.

The best prescriptions or charts can be proposed to the user on the basis of objectives already selected by him before to chart is calculated and displayed.

In order to calculate such charts, the system should preferably have the result available for the patient considered regarding his membrane filtration characteristics (e.g. PET test, miniPET test or PDC test results for Peritoneal Dialysis).

### Detailed description of the invention

The invention is discussed below in a more detailed way with illustrated examples.

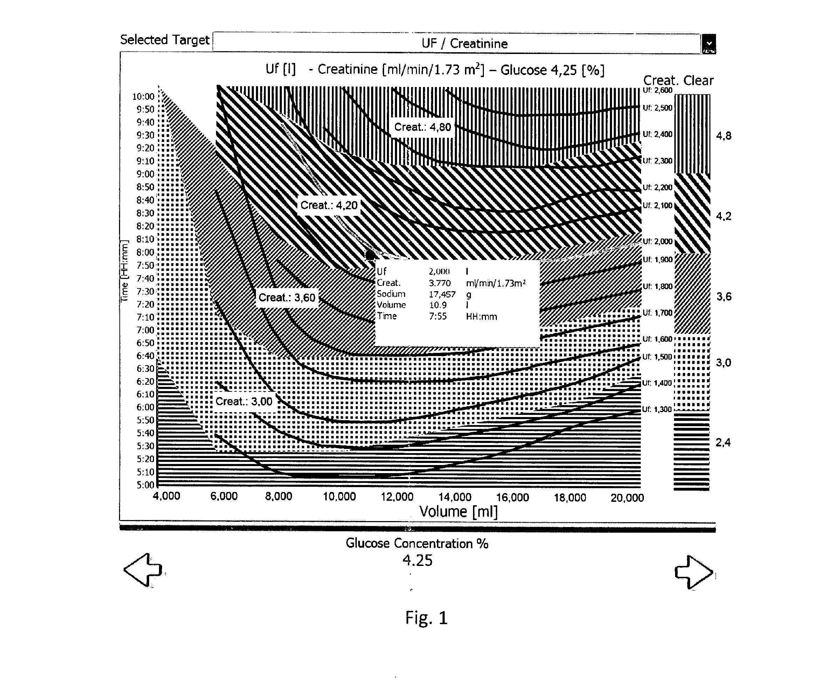

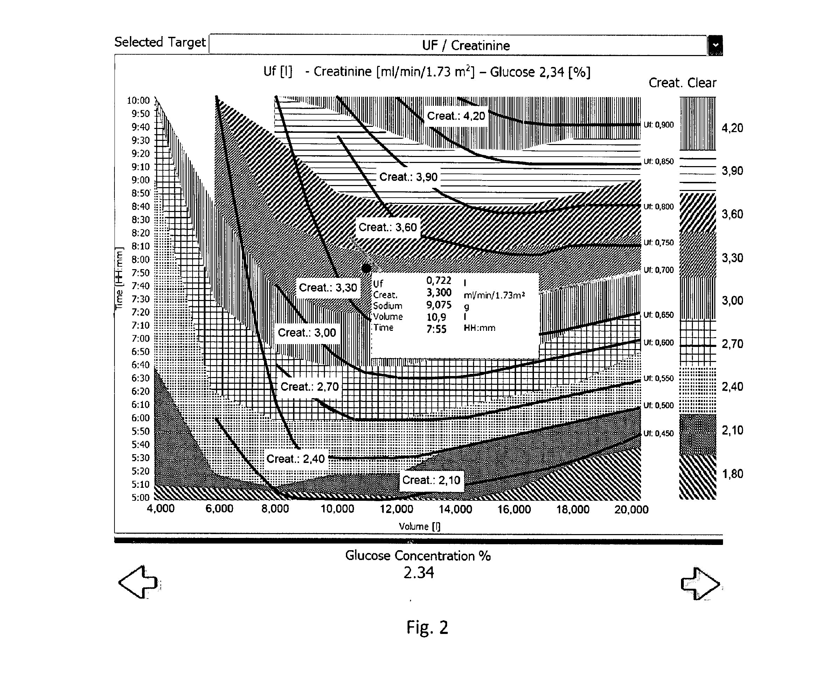

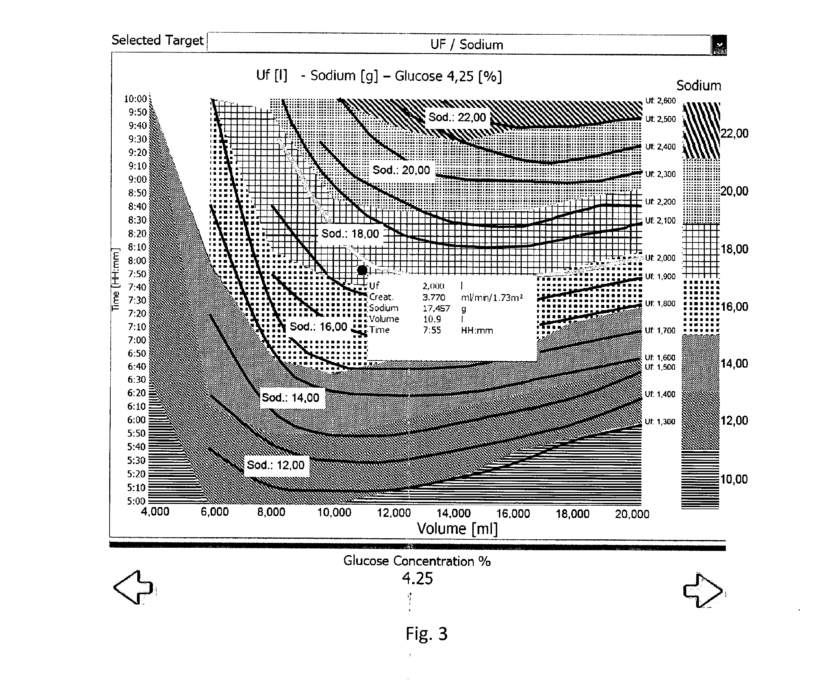

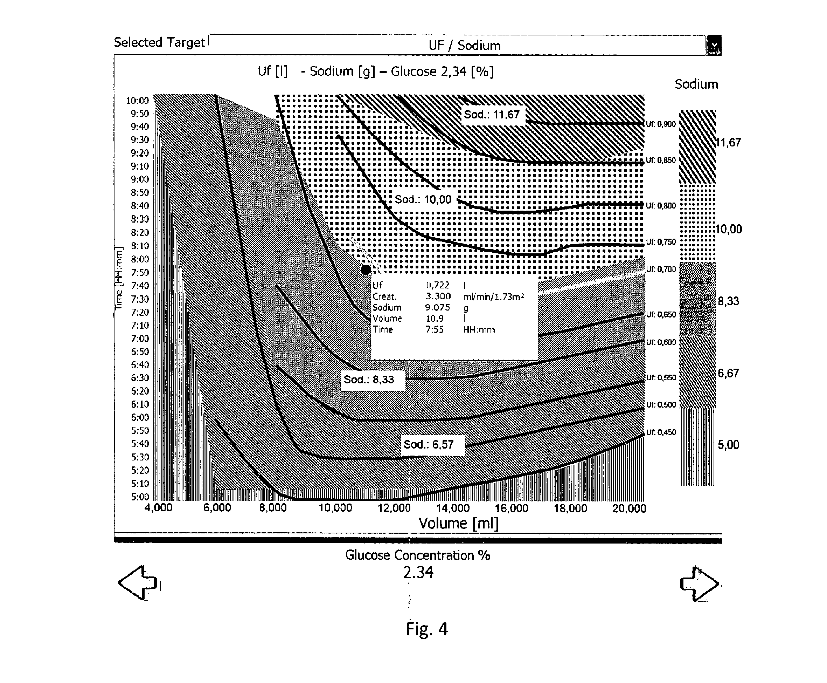

Figures 1 to 4 show examples of screen shots of a system according to the invention wherein the prescription is displayed on a map consisting of an orthogonal bi-dimensional graph. In such graph, the Ultrafiltration is indicated with isolines, each of them representing a fixed result obtained for different peritoneal dialysis volumes and treatment times, based on a given average glucose concentration. In addition (in grayscale on the figures) background colors may be used in a way as to be each indicative of regions representing e.g. different sodium removal value ranges or different ranges of creatinine clearance.

The figures more exactly provide the following information :
Figure 1 shows ultrafiltration isolines with a background representing the creatinine clearance for a fixed glucose concentration of 4.25 %.
Figure 2 shows ultrafiltration isolines with a background representing the creatinine clearance for a fixed glucose concentration of 2.34 %.
Figure 3 shows ultrafiltration isolines with a background representing the sodium removal for a fixed glucose concentration of 4.25 %.
Figure 4 shows ultrafiltration isolines with a background representing the sodium removal for a fixed glucose concentration of 2.34 %.

With such an information, the prescriber can easily select one isoline corresponding to the Ultrafiltration required and further select, within the background colored region the preferred or acceptable one (several regions being acceptable in certain cases), the amount of fluid and duration best corresponding to the patient needs. Since this prescription method uses the membrane characteristics of the patient, the values obtained are representative of the same result which would have been obtained in a state of the art fashion, on an empiric basis, but in only one step taking into consideration all values displayed (treatment time, concentration of glucose, sodium removal and total volume of fluid used), rather than in a step by step empiric manner using simulation tools available. Not only is the prescriber able to reach the best objective in the minimum of time, but he also is able to select the best compromise which, otherwise, would hardly be reached and consequently would only result in an approximation.

The same method of treatment selection can also be used, based on specific parameters relevant therefore, in the case of an hemodialysis treatment based on patient specific targets to be reached.

## Claims

1. Method for prescribing a dialysis treatment comprising the following steps :
- collecting patient specific data,
- determining at least one target,
- defining a series of values of the type [V;t] which allow to achieve said target wherein V represents the volume of dialysate used and t the duration of treatment, based on said patient specific data,
- displaying said series of values on a map.

2. Method according to claim 1, whereby the dialysis is a peritoneal dialysis.

3. Method according to claim 2 wherein said target is taken from the followings: Ultra-filtration, sodium removal or glucose absorption.

4. Method according to claim 2 wherein said target is a clearance of a solute taken from the followings: creatinine, urea or microglobuline.

5. Method according to one of the previous claims wherein said series of values is displayed in the form of a continuous curve in an orthogonal bi-dimensional graph.

6. Method according to claim 5 including the displaying of several series of a values on a map, each series corresponding to a specific target value and defining an isoline on said graph.

7. Method according to one of the previous claims wherein the patient specific data are obtained by a test, such as PET, PDC miniPET or other dialysis tests able to calculate the patient peritoneal membrane characteristics.

8. Method according to one of the previous claims wherein additional information related to at least another patient specific parameter are provided on the map, for instance in the background.

9. Method according to claim 8, wherein said other parameter is taken from the followings : sodium removal, glucose absorption, ultra-filtration, protein intake, microglobuline clearance, effect on diet and kidney function.

10. Peritoneal dialysis prescription system comprising the following elements :
- first processing means for entering patient specific data,
- second processing means for selecting a target,
- third processing means for defining a series of values of the type [V;t] which allow to achieve said target wherein V represents the volume of dialysate used and t the duration of treatment,
- display means adapted for displaying said series of values on a map.

11. Peritoneal dialysis prescription system according to claim 10 wherein said display means are adapted to represent said series of values in the form of a continuous curve in an orthogonal bi-dimensional graph.

12. Peritoneal dialysis prescription system according to claim 11 wherein said display means are adapted to represent several series of values on a map, each series corresponding to a specific target and defining an isoline on said graph.

13. Peritoneal dialysis prescription system according to claim 11 or 12 wherein said target is taken from the followings: Ultra-filtration, sodium removal or glucose absorption.

14. Peritoneal dialysis prescription system according to claim 11 or 12 wherein said target is a clearance of a solute taken from the followings:
creatinine, urea or microglobuline.

15. Peritoneal dialysis prescription system according to one of the previous claims 11 to 14 wherein the patient specific data are obtained by a test, such as PET, PDC miniPET or other dialysis tests able to calculate the patient membrane characteristics.

16. Peritoneal dialysis prescription system according to one of the previous claims 11 to 15 wherein additional information related to at least another patient specific parameter are provided on the map, for instance in the background.

17. Peritoneal dialysis prescription system according to claim 16 wherein said other parameter is taken from the followings : sodium removal, glucose absorption, ultra-filtration, protein intake, microglobuline clearance, effect on diet and kidney function.

18. Peritoneal dialysis prescription system according to one of the previous claims 11 to 17 furthermore comprising display means for displaying the treatment parameters and the expected results for any of said series of values.

19. Peritoneal dialysis prescription system according to one of the previous claims 11 to 18 furthermore comprising automatic parameter recording means which are adapted to automatically record the selected treatment parameters.
